# FASCICULE DE BREVET EUROPEEN

(11) **EP 1 046 398 B1**
(45) Date de publication et mention de la délivrance du brevet: **02.11.2005**
(21) Numéro de dépôt: 00400924.7
(22) Date de dépôt: 04.04.2000
(51) Int. Cl.: A61K 35/78, A61K 7/26, A61P 1/02

(54) **Préparation d'un médicament contenant des composés polyphénoliques de type catéchique ou flavonyliques d'origine végétale, en particulier pour le traitement des gingivites**
Zubereitung eines Arzneimittels enthaltend Catechich- oder Flavonylisch-ähnliche Polyphenolverbindungen aus Pflanzen, insbesondere für die Behandlung von Gingivitis
Preparation of a medicament containing polyphenol compounds of catechic or flavonylic type from plants, especially for the treatment of gingivitis

(30) Priorité: 21.04.1999 FR 9905028
(43) Date de publication de la demande: 25.10.2000
(73) Titulaire: BERKEM, 24680 Gardonne (FR)
(72) Inventeur: Fahy, Lucien, 24520 Lamonzie-Montastruc (FR); Bourges-Sevenier, Cédric, 24130 Prigonrieux (FR)
(74) Mandataire: Abello, Michel

(56) Documents cités:
- WO-A-94/29404
- FR-A- 2 723 943
- PATENT ABSTRACTS OF JAPAN vol. 013, no. 302 (C-616), 12 juillet 1989 (1989-07-12) & JP 01 090124 A (TAIYO KAGAKU CO LTD), 6 avril 1989 (1989-04-06)

## Description

La présente invention concerne la préparation d'un médicament contenant des composés polyphénoliques de type catéchique ou flavoniliques d'origine végétale, en particulier pour le traitement des gingivites.

Il est connu que, par extraction de certaines plantes, on peut obtenir des composés polyphénoliques polymérisés ou non, de préférence sous forme d'oligomères. Ces composés peuvent être notamment des acides hydroxycinnamiques, des proanthocyanines et des flavonoïdes. Ces composés polyphénoliques peuvent être extraits de nombreuses plantes ou parties de plantes, telles que l'écorce d'un conifère, en particulier l'écorce de pin, le thé vert, les potentilles, les pépins de raisin ou analogues. Selon la plante ou la partie de la plante dont ils sont extraits, les composés polyphénoliques présents dans l'extrait ou leurs proportions sont variables. Les extraits obtenus peuvent être traités pour isoler des composés polyphénoliques relativement purs, mais sont le plus souvent utilisés tels quels, c'est-à-dire que l'on utilise un mélange de composés polyphénoliques.

De façon connue, les composés polyphénoliques d'origine végétale possèdent des propriétés biologiques intéressantes parmi lesquelles on peut citer un effet capteur de radicaux libres, donc antivieillissement, un effet antioxydant, un effet anti-inflammatoire, un effet antimicrobien et antiviral et un effet de filtre solaire. On ne leur connaît aucune toxicité.

Ces composés polyphénoliques peuvent être administrés par voie orale. Mais ils présentent un inconvénient : ils ont un goût astringent très prononcé, qui interdit pratiquement toute utilisation telle quelle par voie orale. Il est donc nécessaire de les présenter sous une forme galénique contenue dans des gélules pour les consommer directement par ingestion orale, le plus souvent accompagnés d'une boisson pour les avaler.

Or, les gélules généralement utilisées sont des gélules de gélose, donc des capsules formées avec un matériau d'origine animale, en particulier d'origine bovine. Il en résulte que certains consommateurs refusent, compte tenu des risques de présence dans la gélose de prions de l'encéphalite bovine (ou maladie de la "vache folle"), d'utiliser des produits en gélules. Il serait possible de préparer des gélules avec des produits d'origine végétale, mais le coût des produits est alors considérablement augmenté.

L'utilisation du produit sous forme encapsulée dans une gélule présente un autre inconvénient. Le produit ne peut avoir aucune action directe au niveau buccal proprement dit pour traiter des affections buccales, en particulier les affections de type inflammatoire telles que des gingivites, puisque la prise d'une gélule ne permet pas de la conserver plus de quelques instants dans la bouche et que le produit encapsulé dans une gélule n'est pas en contact avec la muqueuse de la bouche.

Le Patent Abstract of Japan, vol 013, n° 302 (C-616) (abrégé de JP-01 090124) décrit l'utilisation de polyphénols à OH phénoliques contre *Streptococcus mutans,* principale bactérie à l'origine des caries.

Il est, par ailleurs, connu d'estérifier les composés polyphénoliques d'origine végétale pour améliorer leur stabilité. L'estérification de ces composés est, par exemple, décrite dans FR-A-2 723 943 et dans WO 94/29404.

WO 94 29404 décrit des compositions dérivées de polyphénols utilisées pour leur propriétés anti-radicalaires et anti-oxydantes, en vue de la prévention des inflammations des muqueuses avant irradiation.

FR-A-2 723 943 décrit un extrait oligomère polyphénolique d'origine végétale pour application par voie orale.

On effectue, de préférence, l'estérification avec des acides gras à longues chaînes hydrocarbonées. Dans le cas de l'administration par voie orale, on utilise, de préférence, pour l'estérification, les acides gras les plus répandus parmi les corps gras employés en alimentation humaine, par exemple l'acide palmitique et l'acide stéarique. Après absorption orale, ces esters de composés polyphénoliques peuvent, comme les lipides, subir une hydrolyse enzymatique qui restitue les composés polyphénoliques et les acides gras immédiatement au contact de la muqueuse de la bouche.

Selon la présente invention, on a trouvé que, contrairement aux composés polyphénoliques d'origine végétale sous forme libre, c'est-à-dire dans lequel les fonctions OH phénoliques sont libres, les composés polyphénoliques d'origine végétale estérifiés n'ont aucun goût désagréable. Il n'y a donc aucun inconvénient à les administrer par voie orale sans les enfermer dans une gélule, car l'utilisateur peut les mettre en bouche sans avoir à subir un goût désagréable.

La présente invention a donc pour objet un procédé de préparation d'un médicament à administrer par voie orale, contenant au moins un composé polyphénolique d'origine végétale, caractérisé par le fait que l'on utilise le (ou les) composé(s) polyphénolique(s) sous forme d'au moins un ester, sous une forme galénique permettant un contact direct dudit (ou desdits) ester(s) avec la muqueuse buccale.

Selon l'invention, l'ester (ou les esters) de composé(s) polyphénolique(s) d'origine végétale peut (ou peuvent) être sous forme de poudre, de cachet, de gel, de sirop ou de microdispersion aqueuse. Il n'est pas sous forme de gélule ou de cachet enrobé, car il n'y aurait pas contact direct avec la muqueuse buccale.

L'ester (ou les esters) utilisé(s) est (ou sont), de préférence, choisi(s) dans le groupe formé par les esters de composé(s) polyphénolique(s) d'origine végétale avec au moins un acide gras à longue chaîne hydrocarbonée, par exemple l'acide palmitique et/ou l'acide stéarique.

Le(s) composé(s) polyphénolique(s) est (ou sont), de préférence, extrait(s) de l'écorce d'un conifère, du thé vert ou de pépins de raisin.

Selon un mode de mise en oeuvre préféré de l'invention, la présente invention a pour objet l'utilisation d'au moins un ester de composé(s) polyphénolique(s) d'origine végétale pour la préparation d'un médicament destiné au traitement des gingivites.

L'exemple ci-après, donné à titre d'exemple purement illustratif et non limitatif, permettra de mieux comprendre l'invention.

### EXEMPLE :

### 1) Préparation de l'ester de composé polyphénolique

On réalise une suspension de 100 grammes d'oligomères polyphénoliques extraits d'écorce de pin dans 600 ml de chloroforme. On ajoute à cette suspension 250 ml de triéthylamine et une quantité catalytique de 7 grammes de 4-diméthylaminopyridine. On agite ce mélange mécaniquement sous azote et on introduit lentement 400 ml de chlorure de palmitoyle. Lorsque l'addition est terminée, on agite à température ambiante pendant 12 heures. Après cette période de réaction, on évapore à sec sous pression réduite en maintenant la température au-dessous de 40°C.

On reprend ensuite le résidu avec 1 litre d'un mélange méthanol/eau (9/1). On agite le mélange pendant 1 heure à température ambiante, on écarte le surnageant, on ajoute ensuite 1 litre de méthanol et on poursuit l'agitation à température ambiante pendant 1 heure. On filtre ce mélange et on lave le solide avec de l'acétone puis on le sèche.

Le produit obtenu après séchage se présente sous forme d'une poudre de consistance grasse, insoluble dans les solvants polaires et solubles dans les solvants des graisses.

### 2) On procède alors à une mise en comprimés parfumés au kiwi contenant de 50 mg à 100 mg de composés polyphénoliques.

### 3) Traitement

Un sujet atteint de gingivite a pris 6 comprimés par jour. Il n'a constaté aucun goût désagréable lors de la prise des comprimés. Au bout de 2 jours, les symptômes de la gingivite ont disparu.

## Revendications

1. Utilisation de composé(s) polyphénolique(s) d'origine végétale, à fonctions OH phénoliques estérifiées, pour la préparation d'un médicament destiné au traitement des gingivites.

2. Utilisation de composé(s) polyphénolique(s) d'origine végétale selon la revendication 1, sous une forme galénique permettant un contact direct avec la muqueuse buccale.

3. Utilisation selon la revendication 1 ou 2, **caractérisée par le fait que** l'ester de composé(s) polyphénolique(s) d'origine végétale est au moins un ester d'acide gras à longue chaîne hydrocarbonée.

4. Utilisation selon la revendication 3, **caractérisée par le fait que** l'acide gras est l'acide palmitique ou l'acide stéarique.

5. Utilisation selon l'une des revendications 1 à 4, **caractérisée par le fait que** le(s) composé(s) polyphénolique(s) est (sont) susceptible(s) d'être obtenu(s) par extraction de l'écorce d'un conifère, du thé vert ou des pépins de raisin.

## Patentansprüche

1. Verwendung einer phenolischen Verbindung (oder phenolischer Verbindungen) pflanzlichen Ursprungs mit veresterten phenolischen OH-Funktionen zur Herstellung eines Medikaments zur Behandlung von Gingivitis.

2. Verwendung einer phenolischen Verbindung (oder phenolischer Verbindungen) pflanzlichen Ursprungs nach Anspruch 1 in einer galenischen Form, die einen direkten Kontakt mit der Mundschleimhaut gestattet.

3. Verwendung nach Anspruch 1 oder 2, **dadurch gekennzeichnet, dass** der Ester der phenolischen Verbindung (oder der phenolischen Verbindungen) pflanzlichen Ursprungs wenigstens ein Ester einer Fettsäure mit langer Kohlenwasserstoffkette ist.

4. Verwendung nach Anspruch 3, **dadurch gekennzeichnet, dass** die Fettsäure Palmitinsäure oder Stearinsäure ist.

5. Verwendung nach einem der Ansprüche 1 bis 4, **dadurch gekennzeichnet, dass** die phenolische Verbindung (oder die phenolischen Verbindungen) durch Extraktion von Koniferenrinde, grünem Tee oder Traubenkernen erhältlich ist (oder sind).

## Claims

1. Use of polyphenolic compound(s) of plant origin, containing esterified phenolic OH functions, for the preparation of a medicament for treating gingivitis.

2. Use of polyphenolic compound(s) of plant origin according to Claim 1, in a galenical form allowing direct contact with the oral mucosa.

3. Use according to Claim 1 or 2, **characterized in that** the ester of polyphenolic compound(s) of plant origin is at least one ester of a fatty acid containing a long hydrocarbon chain.

4. Use according to Claim 3, **characterized in that** the fatty acid is palmitic acid or stearic acid.

5. Use according to one of Claims 1 to 4, **characterized in that** the polyphenolic compound(s) may be obtained by extraction of a conifer bark, green tea or grapeseeds.
